(19) Europäisches Patentamt / European Patent Office / Office européen des brevets

(11) **EP 4 467 660 B1**

(12) **EUROPEAN PATENT SPECIFICATION**

(45) Date of publication and mention
of the grant of the patent:
**11.03.2026 Bulletin 2026/11**

(21) Application number: **24176678.1**

(22) Date of filing: **17.05.2024**

(51) International Patent Classification (IPC):
***G16B 40/10*** *(2019.01)*

(52) Cooperative Patent Classification (CPC):
(C-Sets available)
**C12Q 1/6816; C12Q 1/6818; C12Q 1/6837;
G16B 40/10;** G01N 21/6408; G01N 21/6456

(Cont.)

(54) **NUCLEIC ACID SEQUENCE MEASUREMENT APPARATUS, NUCLEIC ACID SEQUENCE MEASUREMENT METHOD, NUCLEIC ACID SEQUENCE MEASUREMENT DEVICE, AND STORAGE MEDIUM**

NUKLEINSÄURESEQUENZMESSVORRICHTUNG, NUKLEINSÄURESEQUENZMESSVERFAHREN, NUKLEINSÄURESEQUENZMESSVORRICHTUNG UND SPEICHERMEDIUM

APPAREIL DE MESURE DE SÉQUENCE D'ACIDE NUCLÉIQUE, PROCÉDÉ DE MESURE DE SÉQUENCE D'ACIDE NUCLÉIQUE, DISPOSITIF DE MESURE DE SÉQUENCE D'ACIDE NUCLÉIQUE ET SUPPORT DE STOCKAGE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **19.05.2023 JP 2023083113**

(43) Date of publication of application:
**27.11.2024 Bulletin 2024/48**

(73) Proprietor: **Yokogawa Electric Corporation
Tokyo 180-8750 (JP)**

(72) Inventors:
• **Miyauchi, Yuki
Tokyo, 180-8750 (JP)**
• **Taguchi, Tomoyuki
Tokyo, 180-8750 (JP)**
• **Kuwata, Masahiro
Tokyo, 180-8750 (JP)**
• **Kobayashi, Nobuyuki
Tokyo, 180-8750 (JP)**

(74) Representative: **Grünecker Patent- und
Rechtsanwälte
PartG mbB
Leopoldstraße 4
80802 München (DE)**

(56) References cited:
**CN-A- 102 713 578        CN-C- 1 173 182
GB-A- 2 435 473         US-A1- 2007 122 816
US-A1- 2012 196 767**

EP 4 467 660 B1

(52) Cooperative Patent Classification (CPC): (Cont.)

C-Sets
**C12Q 1/6816, C12Q 2537/165, C12Q 2565/513;**
**C12Q 1/6818, C12Q 2537/165, C12Q 2565/101,**
**C12Q 2565/549;**
**C12Q 1/6837, C12Q 2537/165, C12Q 2563/107,**
**C12Q 2565/101, C12Q 2565/549**

**Description**

CROSS-REFERENCE TO RELATED APPLICATIONS

The present disclosure relates to a nucleic acid sequence measurement apparatus, a nucleic acid sequence measurement method, a nucleic acid sequence measurement device, and storage medium. Priority is claimed on Japanese Patent Application No. 2023-083113, filed on May 19, 2023,

PRIOR ART

As a method of measuring a target with a specific nucleic acid sequence contained in a sample, a method of using a DNA chip (a detection probe having a complementary sequence to the specific nucleic acid sequence described above is provided on a solid phase surface such as a substrate) is widely known. In this method, a sample containing a target is added to a DNA chip, and the target is measured by using the properties of the target reacted by the detection probe of the DNA chip due to hybridization. With this method, it is possible to measure not only whether the target is included in the sample but also the amount of the target included in the sample.

Japanese Patent No. 5928906 describes a method of measuring a target using a nucleic acid sequence measurement device (a DNA chip) provided with a fluorescent probe to which fluorescent molecules are attached and a quenching probe to which a quenching molecule that quenches the fluorescence of the fluorescent molecules as the detection probe described above. In this method, it is possible to measure a target without adding fluorescent molecules to the target and cleaning the DNA chip (cleaning to remove unreacted target, and the like). GB 2 435 473 discloses a method for manufacturing a gel biochip applicable for immobilizing a biologically active compound and/or a fluorescent colourant.

BACKGROUND OF THE INVENTION

Incidentally, in a nucleic acid sequence measurement device, a variation may occur in light intensity of fluorescence emitted from a spot (a measurement area) where a detection probe is provided. For example, a variation in spot light intensity may occur between manufacturing lots, between devices within a manufacturing lot, between blocks (partition areas in units of a plurality of spots) within a device, and between spots within a block.

If there is such a variation in spot light intensity, there is a possibility that detection sensitivity will decrease or reliability will decrease due to the occurrence of false positives or false negatives. Additionally, the spot light intensity for the nucleic acid sequence measurement device may change over time. It is conceivable that such a change in spot light intensity may also reduce the reliability due to the occurrence of false positives or false negatives.

The present invention has been made in view of the circumstances described above, and a purpose thereof is to provide a nucleic acid sequence measurement apparatus, a nucleic acid sequence measurement method, a nucleic acid sequence measurement program, and a nucleic acid sequence measurement device that can measure a target with higher reliability than before, even if there are variations or changes over time in spot light intensity.

SUMMARY OF THE INVENTION

In order to solve the problems described above, a nucleic acid sequence measurement apparatus according to a first aspect of the present invention includes, in a nucleic acid sequence measurement apparatus (1) that measures a target (TG) with a specific nucleic acid sequence contained in a sample, a detector (12) configured to detect fluorescence emitted from a nucleic acid sequence measurement device (DV), which is provided with a measurement area (SP1) that emits first fluorescence due to a reaction with the target, and a reference area (SP2) that emits second fluorescence regardless of the reaction with the target and a calculator (25) configured to measure the target on the basis of a light intensity obtained by correcting or normalizing a light intensity of the first fluorescence emitted from the measurement area using a light intensity of the second fluorescence emitted from the reference area in the fluorescence detected by the detector.

In the nucleic acid sequence measurement apparatus according to a second aspect of the present invention, in the nucleic acid sequence measurement apparatus according to the first aspect of the present invention, the nucleic acid sequence measurement device may be provided with a plurality of measurement areas and a plurality of reference areas, and the calculator may correct or normalize a spatial distribution of a light intensity of the first fluorescence emitted from the plurality of measurement areas using a light intensity of the second fluorescence emitted from the plurality of reference areas.

In the nucleic acid sequence measurement apparatus according to a third aspect of the present invention, in the nucleic acid sequence measurement apparatus according to the second aspect of the present invention, the calculator may calculate a coefficient indicating a change in light intensity of the second fluorescence along a straight line (LN2) passing through any two or more reference areas based on the light intensity of the second fluorescence emitted from the any two or more of the plurality of reference areas, and correct the spatial distribution of the light intensity of the first fluorescence emitted from the measurement area positioned on the straight line using the coefficient.

In the nucleic acid sequence measurement apparatus according to a fourth aspect of the present invention, in the nucleic acid sequence measurement apparatus according to the third aspect of the present invention, the coefficient may be a coefficient indicating a rate of change in light intensity of the second fluorescence along the straight line, or a coefficient based on a multiple regression analysis or a multivariate analysis of the light intensity of the second fluorescence along the straight line.

In the nucleic acid sequence measurement apparatus according to a fifth aspect of the present invention, in the nucleic acid sequence measurement apparatus according to the first aspect of the present invention, the calculator may use information indicating positions of the plurality of reference areas, information indicating the light intensity of the second fluorescence emitted from the plurality of reference areas, and information indicating a position of the measurement area as explanatory variables, , and correct the light intensity of the first fluorescence emitted from the measurement area using a correction coefficient obtained by substituting the explanatory variables into a learning model learned using the correction coefficient in the measurement area as an objective variable.

In the nucleic acid sequence measurement apparatus according to a sixth aspect of the present invention, in the nucleic acid sequence measurement apparatus according to the first aspect of the present invention, the calculator may correct the light intensity of the first fluorescence emitted from the measurement area based on the light intensity of the second fluorescence emitted from the reference area using a relational expression showing a relationship between a change over time in light intensity of the first fluorescence emitted from the measurement area and a change over time in light intensity of the second fluorescence emitted from the reference area.

In the nucleic acid sequence measurement apparatus according to a seventh aspect of the present invention, in the nucleic acid sequence measurement apparatus according to any one of the first to sixth aspects of the present invention, the calculator may extract the measurement area using a predefined light intensity threshold value (THO), and correct the light intensity threshold value according to the light intensity of the second fluorescence emitted from the reference area.

In the nucleic acid sequence measurement apparatus according to an eighth aspect of the present invention, in the nucleic acid sequence measurement apparatus according to any one of the first to seventh aspects of the present invention, the calculator may notify that there is an abnormality when the light intensity of the second fluorescence emitted from the reference area exceeds a preset upper threshold value or does not exceed a preset lower threshold value.

In the nucleic acid sequence measurement apparatus according to a ninth aspect of the present invention, in the nucleic acid sequence measurement apparatus according to any one of the first to eighth aspects of the present invention, the measurement area and the reference area in the nucleic acid sequence measurement device may be divided into partition areas (BK) in units of a predetermined number, and the detector may include an image acquirer (12a) configured to acquire an image of the partition area.

A nucleic acid sequence measurement method according to another aspect of the present invention is a nucleic acid sequence measurement method that measures a target (TG) with a specific nucleic acid sequence contained in a sample, and includes a calculation step (S15 and S16) of measuring, among fluorescence emitted from a nucleic acid sequence measurement device (DV), which is provided with a measurement area (SP1) that emits first fluorescence due to a reaction with the target, and a reference area (SP2) that emits second fluorescence regardless of the reaction with the target, the target on the basis of light intensity obtained by correcting or normalizing a light intensity of the first fluorescence emitted from the measurement area using light intensity of the second fluorescence emitted from the reference area.

A nucleic acid sequence measurement program according to still another aspect of the present invention causes a computer to execute a calculation step (S15 and S16) of measuring, among fluorescence emitted from a nucleic acid sequence measurement device (DV), which is provided with a measurement area (SP1) that emits first fluorescence due to a reaction with the target, and a reference area (SP2) that emits second fluorescence regardless of the reaction with the target, the target on the basis of light intensity obtained by correcting or normalizing a light intensity of the first fluorescence emitted from the measurement area using a light intensity of the second fluorescence emitted from the reference area.

The nucleic acid sequence measurement device according to the first aspect of the present invention is a nucleic acid sequence measurement device (DV) that is used to measure a target (TG) with a specific nucleic acid sequence contained in a sample and includes a measurement area (SP1) that emits first fluorescence due to a reaction with the target and a reference area (SP2) that emits second fluorescence regardless of the reaction with the target.

In the nucleic acid sequence measurement device according to the second aspect of the present invention, in the nucleic acid sequence measurement device according to the first aspect of the present invention, the measurement area and the reference area are divided into partition areas (BK) in units of a predetermined number.

According to the present invention, even if there are variations or changes over time in the amount of spot light, the target can be measured with higher reliability than before.

Further features and aspects of the present disclosure will become apparent from the following detailed description of exemplary embodiments with reference to the attached drawings.

BRIEF DESCRIPTION OF DRAWINGS

     FIG. 1 is a perspective view which schematically shows an appearance of a nucleic acid sequence measurement device according to an embodiment of the present invention.
     FIG. 2 is a diagram which schematically shows a detection probe of the nucleic acid sequence measurement device used in the embodiment of the present invention.
     FIG. 3 is a block diagram which shows a main configuration of a nucleic acid sequence measurement apparatus according to the embodiment of the present invention.
     FIG. 4 is a flowchart which shows an outline of a nucleic acid sequence measurement method according to the embodiment of the present invention.
     FIG. 5 is a diagram for describing spot extraction processing performed in the embodiment of the present invention.
     FIG. 6 is a flowchart which shows spatial correction processing by normalization in units of blocks in the embodiment of the present invention.
     FIG. 7 is a flowchart which shows spatial correction processing using a reference value in units of blocks according to the embodiment of the present invention.
     FIG. 8 is a flowchart which shows spatial correction processing using assumed reference values in units of blocks in the embodiment of the present invention.
     FIG. 9 is a diagram for describing spatial correction processing within a block when there is no reference value in the embodiment of the present invention.
     FIG. 10 is a diagram for describing spatial correction processing using a reference value within a block in the embodiment of the present invention.
     FIG. 11 is a diagram which shows an example of a change in an amount of spot light over time.

DETAILED DESCRIPTION OF THE INVENTION

Hereinafter, a nucleic acid sequence measurement apparatus, a nucleic acid sequence measurement method, a nucleic acid sequence measurement program, and a nucleic acid sequence measurement device according to embodiments of the present invention will be described in detail with reference to the drawings. Below, an outline of an embodiment of the present invention will first be described, and then details of the embodiment of the present invention will be described.

<Outline>

Embodiments of the present invention make it possible to measure a target with higher reliability than before, even if there are variations in or changes over time the amount of spot light. Variations in the amount of spot light may occur, for example, between manufacturing lots, between devices within a manufacturing lot, between blocks (divided areas each consisting of a plurality of spots) within a device, and between spots within a block. An example of a change in the amount of spot light over time is that the amount of spot light gradually decreases over time.

Here, the nucleic acid sequence measurement device is manufactured through the following processes. First, a process is performed to prepare a solution containing a detection probe used by a nucleic acid sequence measurement device. Next, a process of dispensing (spotting) the prepared solution into spots onto a substrate of the nucleic acid sequence measurement device is performed using a dedicated spotter. Next, a process of evaporating a solvent of the spotted solution, and bonding and immobilizing the detection probe to the substrate is performed. Next, a process of blocking, among binding molecules that bind the detection probe and the substrate, binding molecules on the substrate to which the detection probe is not bound is performed. Then, a process of removing a redundant detection probe that is not immobilized to the substrate by cleaning is performed. The nucleic acid sequence measurement device manufactured through such processes is packaged and stored as appropriate.

Possible causes of variations in spot light intensity of the nucleic acid sequence measurement device are as follows. The following (1) to (7) are causes in the manufacturing process of the nucleic acid sequence measurement device. The following (8) and (9) are causes in the manufacturing process of the nucleic acid sequence measurement device and after the packaged nucleic acid sequence measurement device is opened.

     (1) Variation in binding molecules that bind substrate of nucleic acid sequence measurement device and detection probe
     (2) Variation in preparation of solution containing detection probe
     (3) Variation in amount of discharge of spotter in spotting process
     (4) Unevenness in concentration of detection probe due to evaporation of solution in spotting process
     (5) Unevenness in concentration of detection probe due to evaporation of solution in immobilization process
     (6) Unevenness in bonding between binding molecule and detection probe during immobilization process
     (7) Unevenness in deviation of detection probe immobilized to substrate in blocking process, and the like

(8) Fading of detection probe fluorescent molecules and decomposition of detection probe molecules due to environmental light exposure

(9) Unevenness in hydrolysis due to humidity

For example, if there is a variation in preparation of spotting solutions for each manufacturing lot, there will be a variation in spot light intensity between manufacturing lots. In addition, if there is a variation in amount of discharge during spotting or unevenness in concentration of a detection probe, a variation in spot light intensity will occur between blocks within a device or between spots within a block.

If such a variation in spot light intensity occurs, there is a possibility that reliability will decrease due to an occurrence of false positives or false negatives. Additionally, it is considered that changes over time in spot light intensity may also reduce the reliability due to the occurrence of false positives or false negatives. In addition, if the spot light intensity decreases significantly due to the changes over time, there is a possibility that it will not be possible to identify a spot and measurement will not be able to be performed.

In an embodiment of the present invention, fluorescence emitted from a nucleic acid sequence measurement device, which is provided with a measurement area that emits fluorescence due to a reaction with a target with a specific nucleic acid sequence contained in a sample, and a reference area that emits fluorescence regardless of the reaction with the target, is detected. Then, the target is measured on the basis of a light intensity obtained by correcting or normalizing a light intensity of fluorescence emitted from the measurement area using a light intensity of fluorescence emitted from the reference area among the detected fluorescence. For this reason, even if there is a variation or a change over time in spot light intensity in the measurement area, it is possible to measure the target with higher reliability than before.

[Embodiment]

In the following description, first, a nucleic acid sequence measurement device used in a measurement of a nucleic acid sequence will be described. Subsequently, a nucleic acid sequence measurement apparatus, a nucleic acid sequence measurement method, and a nucleic acid sequence measurement program that measure a nucleic acid sequence using the nucleic acid sequence measurement device described above will be described.

<Nucleic acid sequence measurement device>

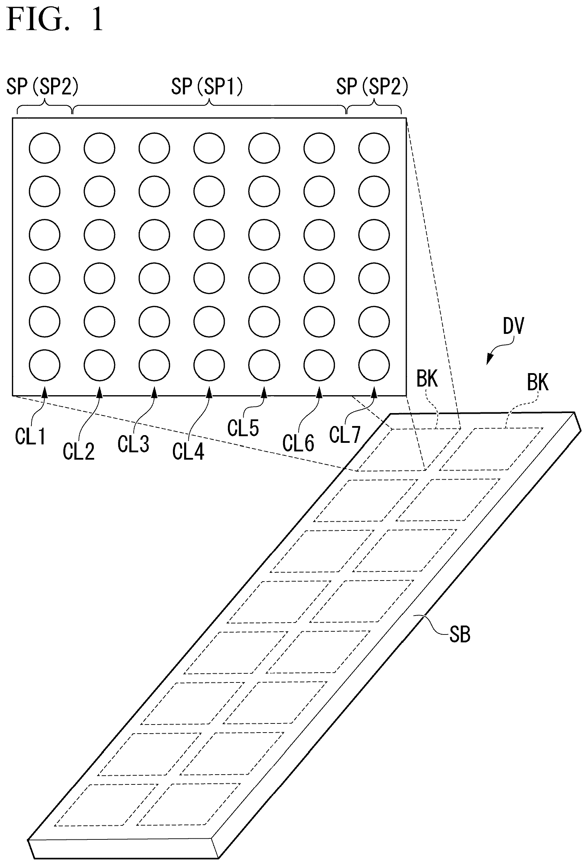

FIG. 1 is a perspective view which schematically shows an appearance of a nucleic acid sequence measurement device according to an embodiment of the present invention. Note that in FIG. 1, a part of the nucleic acid sequence measurement device is shown enlarged. As shown in FIG. 1, the nucleic acid sequence measurement device DV includes, for example, a plurality of spots SP formed on a substrate SB. As the substrate SB, for example, a plate-shaped glass formed into a rectangular shape in a plan view, a single crystal such as silicon, calcium fluoride, and sapphire, a ceramic, a resin material, and the like can be used. Examples of the resin material include cycloolefin polymer (COP), which has excellent optical properties, chemical and thermal stability, cyclic olefin copolymer (COC), polycarbonate, an acrylic resin, a polyethylene resin, and the like. Note that a shape of the substrate SB may be any shape in a plan view.

The spot SP includes a detection probe spot SP1 (a measurement area) and a marker spot SP2 (a reference area). The detection probe spot SP1 is an area in which a detection probe (details will be described below) used to detect a target, which is a measurement target, is fixed. A marker spot SP2 is an area in which a fluorescent probe (details will be described below) is fixed. The marker spot SP2 is provided to correct or normalize variations in spot light intensity in the detection probe spot SP1.

The spot SP is divided into blocks BK (partition areas) each having a predetermined number of units. In the nucleic acid sequence measurement device DV shown in FIG. 1, a total of 672 spots SP are divided into 16 blocks BK in units of a total of 42 spots SP arranged in 6 rows and 7 columns. In an example shown in FIG. 1, six marker spots SP2 are arranged in a first column CL1 and a seventh column CL7 of each block BK, respectively, and six detection probe spots SP1 are arranged in the second column CL2 to the sixth column CL6 of each block BK, respectively.

For the detection probe spots SP1 arranged in the second column CL2 to the sixth column CL6 in each block BK, for example, it is assumed that those arranged in the same column reacted the same target, but those arranged in different columns reacted different targets. Note that all of the detection probe spots SP1 arranged in the second column CL2 to the sixth column CL6 may react the same target. Alternatively, all of the detection probe spots SP1 arranged in the second column CL2 to the sixth column CL6 may react different targets. The number of detection probe spots SP1 that react the same target is arbitrary.

Although FIG. 1 shows an example in which the marker spots SP2 are arranged in the first column CL1 and the seventh column CL7 of each block BK, the arrangement of the marker spots SP2 is not limited to this. For example, only one marker spot SP2 may be provided in each block BK, or a plurality of marker spots SP2 may be provided. Moreover, the marker spot SP2 may be provided with a substrate SB as a reference, with a block BK as a reference, or with a specific area within a block BK as a reference.

An addition of a sample to the nucleic acid sequence measurement device DV is performed for each block BK. In addition, an image acquisition of the nucleic acid sequence measurement device DV is often performed for each block BK.

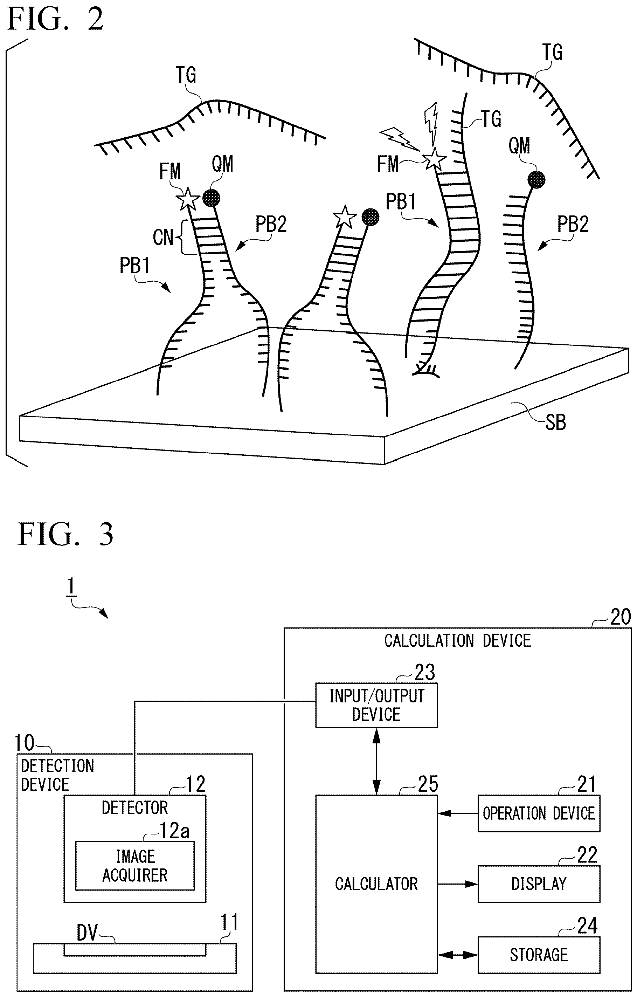

FIG. 2 is a diagram which schematically shows a detection probe of the nucleic acid sequence measurement device used in the embodiment of the present invention. As shown in FIG. 2, the detection probe consists of a fluorescent probe PB1 and a quenching probe PB2 fixed on the substrate SB. The fluorescent probe PB1 is obtained by adding a fluorescent molecule FM to a complementary sequence of a target TG to be measured. The quenching probe PB2 is obtained by adding a quenching molecule QM to a sequence that is at least partially complementary to the complementary sequence described above of the fluorescent probe PB 1.

The target TG is not particularly limited as long as it is a target to be detected in a sample, and examples thereof include nucleic acids such as DNA and RNA, peptides, and proteins. Capture molecules that are specifically bound to the target TG include detection probes that hybridize with nucleic acids, antibodies or antibody fragments that are specifically bound to antigens such as peptides and proteins, aptamers that are specifically bound to nucleic acids, and the like. As the antibody described above, any one of a polyclonal antibody and a monoclonal antibody can be used, but a monoclonal antibody is preferable. Examples of the antibody fragments include $F(ab')_2$, $F(ab)_2$, Fab', Fab, Fv, scFv, variants thereof, and fusion proteins or fusion peptides containing an antibody portion, and the like. Alternatively, the target may be an antibody or an antibody fragment, and the capture molecule may be an antigen such as a peptide or protein that is specifically bound to the antibody or antibody fragment.

Fluorescent molecules FM are not particularly limited as long as they are molecules that generate fluorescence when excited by specific excitation light, but examples thereof include Alexa Fluor (registered trademark) series, ATTO series, Brilliant series, Chromeo (registered trademark) series, Bacteriochlorin series, FAM, TAMRA, Cy dye series, FITC, HiLyte Fluor (registered trademark) series, Rhodamine series, Tide Fluor(registered trademark) series, iFluor (registered trademark) series, DY dye series, B-Phycoerythrin, R-Phycoerythrin, APC, Qdot, PID fluorescent nanoparticles, and the like.

The fluorescent probe PB1 and the quenching probe PB2 are bound so that the fluorescence of the fluorescent molecule FM is quenched by the quenching molecule QM. In the example shown in FIG. 2, the fluorescent probe PB1 and the quenching probe PB2 are bound at a binding portion CN. Here, the fluorescence of the fluorescent molecule FM is quenched by the quenching molecule QM according to principle of quenching by fluorescence resonance energy transfer.

When the target TG is not present, the fluorescent probe PB1 and the quenching probe PB2 are bound at the binding portion CN, and the fluorescent molecule FM and the quenching molecule QM are in a close state. In this state, even if excitation light is irradiated, the fluorescence of the fluorescent molecule FM is quenched by the quenching molecule QM, so that no fluorescence is emitted.

On the other hand, when the target TG is present, as shown in FIG. 2, the fluorescent probe PB1 having a complementary sequence of the target TG dissociates itself from the quenching probe PB2 and binds to target TG. When the target TG is bound to the fluorescent probe PB1, the binding between the fluorescent probe PB1 and the quenching probe PB2 is released, and the fluorescent molecule FM and the quenching molecule QM become separated. In this state, fluorescence is emitted from the fluorescent molecules FM by irradiation with excitation light.

Note that the complementary sequence of the target TG may be provided in the quenching probe PB2. In other words, the quenching probe PB2 may be obtained by adding the quenching molecule QM to the complementary sequence of the target TG, and the fluorescent probe PB1 may also be obtained by adding the fluorescent molecule FM to a sequence that is at least partially complementary to the complementary sequence of quenching probe PB2 described above.

A detection probe in which the fluorescent probe PB1 and the quenching probe PB2 are bound is fixed to the detection probe spot SP1 shown in FIG. 1. For this reason, the detection probe spot SP1 emits fluorescence (first fluorescence) by reacting with the target TG. On the other hand, in the marker spot SP2 shown in FIG. 1, the quenching probe PB2 is not fixed, and only the fluorescent probe PB1 is fixed. For this reason, the marker spot SP2 emits fluorescence (second fluorescence) regardless of whether it reacts with the target TG.

<Nucleic acid sequence measurement apparatus>

FIG. 3 is a block diagram which shows a main configuration of a nuclear acid sequence measurement device according to the embodiment of the present invention. As shown in FIG. 3, the nucleic acid sequence measurement apparatus 1 according to the present embodiment includes a detection device 10 and a calculation device 20, and uses the nucleic acid sequence measurement device DV described using FIGS. 1 and 2 to perform measurement of the target TG contained in a sample.

The detection device 10 includes a stage 11 and a detector 12, and detects fluorescence emitted from the nucleic acid sequence measurement device DV. The stage 11 is a stage configured to be able to place the nucleic acid sequence measurement device DV. It is desirable that the stage 11 is provided with a function to adjust a temperature of the placed nucleic acid sequence measurement device DV, and it is desirable that it is configured to be able to stir a sample by vibrating or rotating the nucleic acid sequence measurement device DV.

The detector 12 irradiates the nucleic acid sequence measurement device DV with excitation light and detects fluorescence emitted from the nucleic acid sequence measurement device DV. This detector 12 includes an excitation light source (not shown) and an image acquirer 12a. An excitation light source (not shown) emits excitation light to irradiate the nucleic acid sequence measurement device DV. The excitation light emitted from the excitation light source is applied to, for example, each block BK shown in FIG. 1. As the excitation light source (not shown), a laser light source that emits laser light with a single wavelength or expanded light thereof, a light emitting diode (LED), a lamp that emits white light, and a light source consisting of a combination of an LED and a wavelength filter, and the like can be used.

The image acquirer 12a includes a solid-state imaging device such as a charge coupled device (CCD) or a complementary metal oxide semiconductor (CMOS), and acquires an image (a two-dimensional image) of the nucleic acid sequence measurement device DV. The image acquirer 12a acquires, for example, an image for each block BK shown in FIG. 1.

The calculation device 20 performs measurement of the target TG on the basis of a result of detection by the detection device 10. Specifically, the calculation device 20 measures whether the target TG is contained in the sample added to the nucleic acid sequence measurement device DV as well as an amount of the target contained in the sample. This calculation device 20 includes an operation device 21, a display 22, an input/output device 23, a storage 24, and a calculator 25.

The operation device 21 includes, for example, an input device such as a keyboard or a pointing device, and outputs an instruction to the calculator 25 (an instruction to the calculation device 20) according to an operation by a user using the calculation device 20. The display 22 includes, for example, a display device such as a liquid crystal display, and displays various types of information output from the calculator 25. Note that the operation device 21 and the display 22 may be physically separated, or may be physically integrated, like a touch panel type liquid crystal display device that has both a display function and an operation function.

The input/output device 23 is connected to the detector 12 of the detection device 10 and inputs or outputs various types of data to or from the detector 12. For example, the input/output device 23 outputs control data for causing the detector 12 to emit excitation light from an excitation light source (not shown). The input/output device 23 also outputs control data for detection of the detector 12, receives a result of the detection (image data acquired by the image acquirer 12), and outputs it to the calculator 25.

The storage 24 includes an auxiliary storage device such as a hard disk drive (HDD) or a solid-state drive (SSD), and stores various types of data. For example, the storage 24 stores image data output from the detector 12, various types of data required for calculations by the calculator 25, data indicating results of the calculations by the calculator 25, and other data. Note that the storage 24 may store, for example, a program that realizes functions of the calculator 25.

The calculator 25 causes the storage 24 to store the image data output from the input/output device 23. In addition, the calculator 25 reads the image data stored in the storage 24, performs image processing on the image data, and performs measurement of the target TG contained in the sample added to the nucleic acid sequence measurement device DV. Specifically, after hybridization, the calculator 25 corrects or normalizes a spot light intensity in the detection probe spot SP1 in a block BK of the nucleic acid sequence measurement device DV using a spot light intensity in the marker spot SP2. Then, the calculator 25 measures the target TG on the basis of the corrected or normalized spot light intensity. Details of the processing performed by the calculator 25 will be described below.

The functions of the calculator 25 may be realized in software by, for example, a central processing unit (CPU) or a micro processing unit (MPU) reading and executing a program stored in the storage 24. Alternatively, the functions of the calculator 25 may be realized using hardware such as a field programmable gate array (FPGA), large scale integration (LSI), or an application specific integrated circuit (ASIC).

<Nucleic acid sequence measurement method>

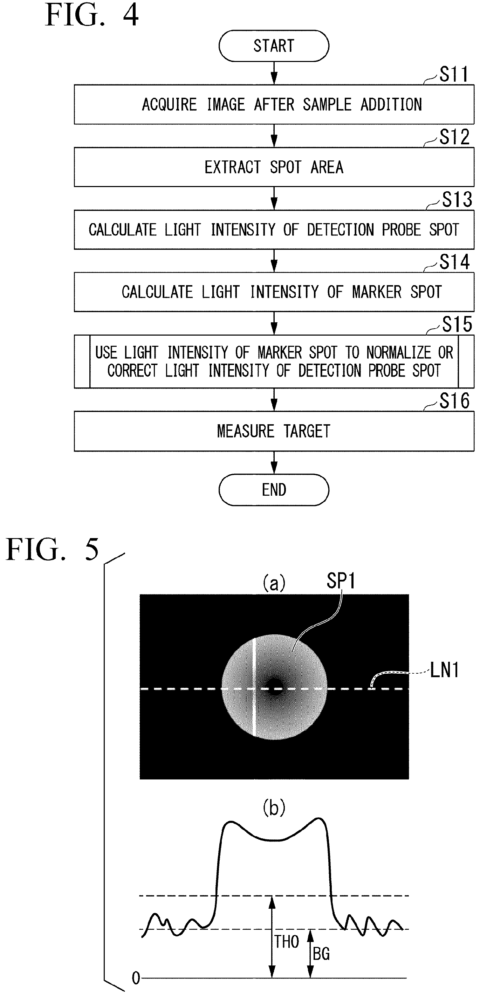

FIG. 4 is a flowchart which shows an outline of a nucleic acid sequence measurement method according to the embodiment of the present invention. Note that a sample including the target TG is prepared before processing of the flowchart shown in FIG. 4 is started. Moreover, work is performed to prepare the nucleic acid sequence measurement device DV described using FIGS. 1 and 2 in a stage 11 of the nucleic acid sequence measurement apparatus 1. When the preparation and work are completed, for example, a sample is added to one of a plurality of blocks BK (hereinafter referred to as a "target block BK0") of the nucleic acid sequence measurement device DV.

When a sample is added to the nucleic acid sequence measurement device DV, the detector 12 first performs processing of acquiring an image after the sample is added (step S11: detection step). Specifically, after a period of time during which hybridization is considered to have sufficiently progressed has elapsed, the image acquirer 12a performs processing of acquiring an image of the target block BK0. The image acquired in step S11 is output from the detector 12 to the calculation device 20. The image output from the detector 12 to the calculation device 20 is stored in the storage 24 of the calculation device 20.

Here, the period of time during which hybridization is considered to have sufficiently progressed varies depending on properties of the nucleic acid sequence measurement device DV (properties of the fluorescent probe PB1 and the quenching probe PB2) and the amount of the target TG contained in the sample. For this reason, it is desirable to predetermine the period of time described above by conducting, for example, an experiment in advance.

Next, the calculator 25 of the calculation device 20 reads the image stored in the storage 24 to perform image processing, and performs processing of extracting a spot (step S12: detection step). Specifically, image processing is performed on the image acquired in step S11 (the image of the target block BK0) to perform processing of extract a spot area (an area estimated to be an image of a spot SP).

FIG. 5 is a diagram for describing spot extraction processing performed in the embodiment of the present invention. FIG. 5(a) is a diagram which shows an image of one detection probe spot SP1 and its surroundings, and FIG. 5(b) is a diagram which shows light intensity distribution along a line LN1 in FIG. 5(a). Fluorescence is emitted from the detection probe spot SP1 in the target block BK0. For this reason, as shown in FIG. 5(a), an image of the detection probe spot SP1 is brighter than the surroundings of the detection probe spot SP1. In addition, the light intensity distribution along the line LN1 in FIG. 5(a) is high inside the detection probe spot SP1 and low outside the detection probe spot SP1, as shown in FIG. 5(b).

As shown in FIG. 5(b), a background light intensity BG and a background noise are present outside the detection probe spot SP1. If light intensity of fluorescence emitted from the detection probe spot SP1 is about the same as the background light intensity BG, the detection probe spot SP1 cannot be extracted. In the present embodiment, a threshold value TH0 (a light intensity threshold), which is set to a larger light intensity than the background light intensity BG, is defined. The calculator 25 of the calculation device 20 performs processing of extracting an area where a light intensity exceeding the threshold value TH0 is emitted as the detection probe spot SP1. Note that although an example in which the detection probe spot SP1 is extracted has been described here, the marker spot SP2 is also extracted by the same processing.

Next, processing of calculating the light intensity of the detection probe spot SP1 is performed by the calculator 25 of the calculation device 20 (step S13: detection step). Specifically, a spot area extracted in step S12 is sorted into an area of the detection probe spot SP1 and an area of the marker spot SP2 on the basis of the position within the imaged target block BK0. Then, for each of the sorted areas of the detection probe spot SP1, processing of calculating an average gradation value of each pixel forming the image of the detection probe spot SP1 as the light intensity of the detection probe spot SP1 is performed.

Moreover, processing of calculating light intensity of the marker spot SP2 is performed by the calculator 25 of the calculation device 20 (step S14: detection step). Specifically, processing of calculating the average gradation value of each pixel forming the image of the marker spot SP2 as the light intensity of the marker spot SP2 is performed on each area of the marker spot SP2 sorted in the processing of step S13. Note that in FIG. 4, the processing in step S13 and the processing in step S14 are separated for ease of understanding, but these pieces of processing may be performed simultaneously.

Subsequently, the calculator 25 of the calculation device 20 uses the light intensity of the marker spot SP2 to perform processing of normalizing or correcting the light intensity of the detection probe spot SP1 (step S15: calculation step). For example, the calculator 25 corrects or normalizes the spatial distribution of the light intensity of fluorescence emitted from the plurality of detection probe spots SP1 provided in the target block BK0 using the light intensity of fluorescence emitted from the plurality of marker spots PS2 provided in the target block BK0. Note that there are several types of processing for normalizing or correcting the light intensity of the detection probe spot SP1 using the light intensity of the marker spot SP2, so that details of the processing of step S15 will be described below.

When the processing described above is completed, the calculator 25 of the calculation device 20 performs the processing of measuring the target TG contained in the sample (step S16: calculation step). Specifically, the calculator 25 determines whether the target TG is contained in the sample added to the nucleic acid sequence measurement device DV. In addition, the amount of the target contained in the sample is measured by the calculator 25 on the basis of the normalized or corrected light intensity of the detection probe spot SP1. In this manner, measurement of the target TG contained in the sample is performed.

<Processing of step S15>

The processing in step S15 shown in FIG. 4 is broadly divided into processing of normalizing or correcting a spatial variation in the spot light intensity (hereinafter referred to as spatial correction processing) and processing of normalizing or correcting changes over time in the spot light intensity (hereinafter referred to as temporal correction processing). Hereinafter, these spatial correction processing and temporal correction processing will be described in order.

<<Spatial correction processing>>

Spatial variations in the spot light intensity occur, for example, between manufacturing lots, between devices for nucleic acid sequence measurement DV within a manufacturing lot, between blocks BK within the nucleic acid sequence measurement device DV, and between spots SP within a block BK. In the following description, spatial correction processing in units of block BK and spatial correction processing within a block BK will be described as examples.

- Spatial correction processing in units of block BK

(a) Spatial correction processing by normalization

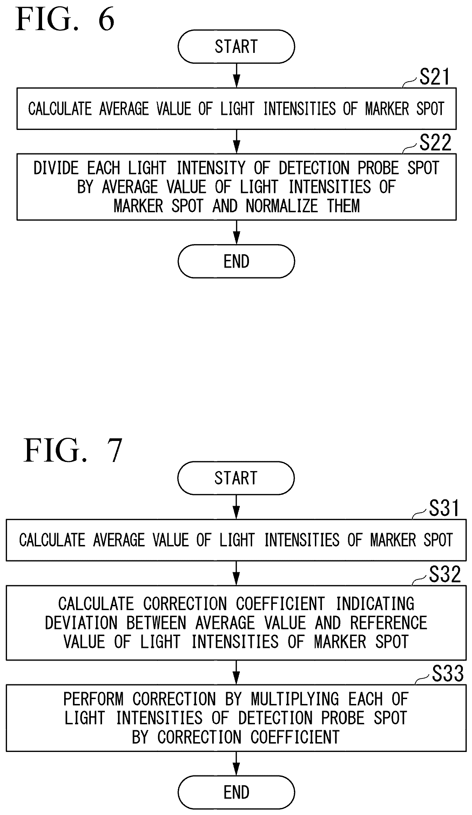

FIG. 6 is a flowchart which shows spatial correction processing using normalization in units of blocks in the embodiment of the present invention. When the processing is started, first, the calculator 25 of the calculation device 20 performs processing of calculating an average value of the light intensities of the marker spot SP2 (step S21). Specifically, processing of calculating an average value of light intensities of a total of 12 marker spots SP2 in the target block BK0, which is calculated in step S14 shown in FIG. 4, is performed. Note that the total of 12 marker spots SP2 in the target block BK0 are the marker spots SP2 arranged in the first column CL1 and the seventh column CL7 of a block BK shown in FIG. 1.

Next, the calculator 25 of the calculation device 20 performs processing of dividing each light intensity of the detection probe spot SP1 by the average value of the light intensities of the marker spot SP2 (step S22). Specifically, processing of dividing each of light intensities of a total of 30 detection probe spots SP1 in the target block BK0, calculated in step S13 shown in FIG. 4, by the average value of the light intensities of the marker spot SP2 is performed. Note that the total of 30 detection probe spots SP1 in the target block BK0 are the detection probe spots SP1 arranged in the second column CL2 to the sixth column CL6 of a block BK shown in FIG. 1. By performing the processing, each light intensity of the detection probe spot SP1 is normalized by the average value of the light intensities of the marker spot SP2.

When the processing described above is completed, in step S16 shown in FIG. 4, the calculator 25 of the calculation device 20 performs the processing of measuring the target TG contained in the sample on the basis of the normalized light intensity of the detection probe spot SP1. In the processing, using a determination threshold value set for spatial correction processing by normalization (hereinafter referred to as a first determination threshold value), it is determined whether the target TG is contained in the sample added to the nucleic acid sequence measurement device DV.

(b) Spatial correction processing using reference value

FIG. 7 is a flowchart which shows spatial correction processing using a reference value in units of blocks in the embodiment of the present invention. Here, the reference value is a value indicating a light intensity that is a reference of the marker spot SP2 of the nucleic acid sequence measurement device DV. This reference value is the average value, a median value, or the like of the light intensities of the marker spot SP2 obtained in a state in which a sample is added to the nucleic acid sequence measurement device DV manufactured in advance in a plurality of manufacturing lots and hybridization is performed (wet state). Note that when the reference value is calculated, it is preferable to use the obtained light intensities of the marker spot SP2, excluding those with significantly different light intensities (outliers). Note that the reference value is, for example, stored in advance in the storage 24 of the calculation device 20 in the nucleic acid sequence measurement apparatus 1 shown in FIG. 3.

When processing of the flowchart shown in FIG. 7 is started, first, the processing of calculating the average value of the light intensities of the marker spot SP2 is performed by the calculator 25 of the calculation device 20 (step S31). Specifically, similar to the processing of step S21 shown in FIG. 6, processing of calculating the average value of the light intensities of a total of 12 marker spots SP2 in the target block BK0 is performed.

Next, the calculator 25 of the calculation device 20 performs processing of calculating a correction coefficient indicating a deviation between the average value and the reference value of the light intensities of the marker spot SP2 (step S32). Specifically, processing of calculating a correction coefficient is performed by dividing the average value of the light intensities of the marker spot SP2 calculated in step S31 by the reference value.

Subsequently, processing of multiplying each of the light intensities of the total of 30 detection probe spots SP1 in the target block BK0, calculated in step S13 shown in FIG. 4, by the correction coefficient calculated in step S32 is performed (step S33). By performing this processing, each light intensity of the detection probe spot SP1 is corrected using a correction coefficient that indicates a deviation between the average value and the reference value of the light intensities of the marker spot SP2.

When the processing described above is completed, in step S16 shown in FIG. 4, the calculator 25 of the calculation device 20 performs the processing of measuring the target TG contained in the sample on the basis of the corrected light intensity of the detection probe spot SP1. In this processing, it is determined whether the target TG is contained in the sample added to the nucleic acid sequence measurement device DV using a determination threshold value set for the spatial correction processing using a reference value. Note that the determination threshold value set for the spatial correction processing using a reference value (hereinafter referred to as a second determination threshold value) is different from the first determination threshold value described above.

(c) Spatial correction processing using assumed reference value

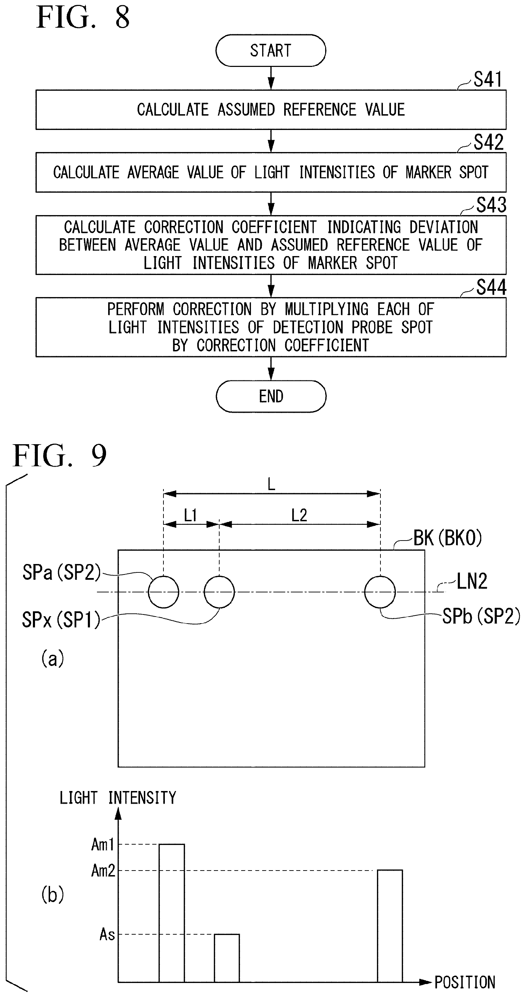

FIG. 8 is a flowchart which shows spatial correction processing using an assumed reference value in units of blocks in the embodiment of the present invention. Here, the assumed reference value is a value indicating the light intensity that serves as a reference for the marker spot SP2 of the nucleic acid sequence measurement device DV, similarly to the reference value described above. However, a difference is that the reference value described above is a value that is calculated in advance, whereas the assumed reference value is a value that is calculated each time the nucleic acid sequence measurement device DV is used.

When the assumed reference value is used, for example, a ratio (hereinafter referred to as a state correction value) of the average value, the median value, or the like of the light intensity of the marker spot SP2 obtained in a state (a dry state) in which no sample is added to the nucleic acid sequence measurement device DV manufactured in advance in a plurality of manufacturing lots to the average value, the median value, or the like of the light intensities of the marker spot SP2 obtained in a wet state is calculated. Note that this state correction value is, for example, stored in advance in the storage 24 of the calculation device 20 in the nuclear acid sequence measurement device 1 shown in FIG. 3.

In addition, for each nucleic acid sequence measurement device DV to be shipped, the light intensities of the marker spot SP2 are acquired in the dry state, and the average value, the median value, or the like of the acquired light intensity (hereinafter referred to as a dry value) is calculated. Note that the light intensities of the marker spot SP2 can be acquired in the dry state without destroying the nucleic acid sequence measurement device DV, so that it is possible to ship the nucleic acid sequence measurement device DV for which the light intensities of the marker spot SP2 are acquired. The dry value calculated for each nucleic acid sequence measurement device DV is associated with an identifier of the nucleic acid sequence measurement device DV, and is stored in the storage 24 of the calculation device 20 in the nucleic acid sequence measurement apparatus 1 shown in FIG. 3 in advance. The dry value may be stored in an electronic tag attached to the nucleic acid sequence measurement device DV to be shipped, or printed as a barcode on the tag attached to the nucleic acid sequence measurement device DV to be shipped.

When processing of the flowchart shown in FIG. 8 is started, first, the calculator 25 of the calculation device 20 performs processing of calculating an assumed reference value (step S41). Specifically, for example, processing of reading the state correction value stored in the storage 24 and the dry value of the nucleic acid sequence measurement device DV installed in the stage 11 from the storage 24, and calculating an assumed reference value by multiplying the read state correction value and dry value is performed. Note that the assumed reference value can also refer to as a value indicating the light intensity that is assumed to be a reference for the marker spot SP2 in a wet state, assume based on the light intensities of the marker spot SP2 obtained in the dry state.

Next, the calculator 25 of the calculation device 20 performs processing of calculating the average value of the light intensities of the marker spot SP2 (step S42). Specifically, similar to the processing in step S21 shown in FIG. 6 and step S31 shown in FIG. 7, processing of calculating the average value of the light intensities of the total of 12 marker spots SP2 in the target block BK0 is performed.

Subsequently, the calculator 25 of the calculation device 20 performs processing of calculating a correction coefficient indicating the deviation between the average value of the light intensities of the marker spot SP2 and the assumed reference value (step S43). Specifically, processing of calculating a correction coefficient by dividing the average value of the light intensities of the marker spot SP2 calculated in step S42 by the assumed reference value.

Then, processing of multiplying each of the light intensities of the total of 30 detection probe spots SP1 in the target block BK0, calculated in step S13 shown in FIG. 4, by the correction coefficient calculated in step S43 is performed (step S44). By performing this processing, each light intensity of the detection probe spot SP1 is corrected using a correction coefficient that indicates the deviation between the average value of the light intensities of the marker spot SP2 and the assumed reference value.

When the processing described above is completed, in step S16 shown in FIG. 4, the calculator 25 of the calculation device 20 performs processing of measuring the target TG contained in the sample on the basis of the corrected light intensity of the detection probe spot SP1. In this processing, it is determined whether the target TG is contained in the sample added to the nucleic acid sequence measurement device DV using a determination threshold value similar to the second determination threshold value described above.

As described above, the assumed reference value is calculated for each nucleic acid sequence measurement device DV using the state correction value calculated in advance and the dry value of the nucleic acid sequence measurement device DV. As a result, even if a manufacturing variation of the nucleic acid sequence measurement device DV is large and cannot be corrected using the reference value described above, it is possible to correct each nucleic acid sequence measurement device DV.

- Spatial correction processing within block BK

Spatial correction processing in a block BK is basically performed using a light intensity of fluorescence emitted from any two or more marker spots SP2 among the plurality of marker spots SP2 in the block BK (the target block BK0). Specifically, a coefficient indicating a change in light intensity of fluorescence along a straight line passing through any two or more marker spots SP2 in the block BK (the target block BK0) is calculated, and the spatial distribution of the light intensity of fluorescence emitted from the detection probe spot SP1 positioned on the straight line is corrected using the coefficient described above.

Here, the coefficient described above may be a coefficient indicating a rate of change in light intensity of fluorescence emitted from the marker spot SP2 along the straight line described above. Alternatively, the coefficient described above may be a coefficient based on a multiple regression analysis or multivariate analysis of the light intensity of fluorescence emitted from the marker spot SP2 along the straight line described above.

In addition, when correction is performed using a coefficient based on the multiple regression analysis, for example, a marker spot light intensity at arbitrary coordinates in the image of the target block BK0 is set as an objective variable y, a horizontal direction coordinate in the image is set as an explanatory variable x1, and a vertical coordinate within the image is set as an explanatory variable x2. Then, partial regression coefficients $\alpha 1$ and $\alpha 2$ of the explanatory variables x1 and x2, and a constant C are calculated based on the plurality of marker spot light intensities. A predicted value of a marker spot light intensity can be calculated by substituting arbitrary coordinates into the following equation (1). The spot light intensity can be corrected using a light intensity ratio between the predicted value of this marker spot light intensity and the reference value described above as a coefficient.

$$y = \alpha 1 \times x1 + \alpha 2 \times x2 + C \ldots (1)$$

A specific example of spatial correction processing will be described below.

(a) Spatial correction processing when there is no reference value

FIG. 9 is a diagram for describing spatial correction processing within a block when there is no reference value in the embodiment of the present invention. FIG. 9(a) is a diagram which shows a positional relationship of spots in a block BK (the target block BK0), and FIG. 9(b) is a diagram which shows light intensities of the spots shown in FIG. 9(a). Note that, in FIG. 9, among the plurality of spots SP arranged in a block BK shown in FIG. 1, only three spots SP arranged on a straight line LN2 extending in a row direction are shown.

In FIG. 9(a), the marker spot SP2 arranged in the first column CL1 of a block BK shown in FIG. 1 is shown as a marker spot SPa, and the marker spot SP2 arranged in the seventh column CL7 is shown as a marker spot SPb. Moreover, one of the detection probe spots SP1 arranged in the second column CL2 to the sixth column CL6 of the block BK shown in FIG. 1 is shown as a detection probe spot SPx. In addition, a distance between the marker spot SPa and the marker spot SPb is L, a distance between the marker spot SPa and the detection probe spot SPx is L1, and a distance between the detection probe spot SPx and the marker spot SPb is L2.

As shown in FIG. 9(b), it is assumed that the light intensity obtained from the marker spots SPa arranged on the straight line LN2 is Am1, and the light intensity obtained from the marker spot SPb is Am2. In addition, it is also assumed that the light intensity obtained from the detection probe spot SPx is As. The calculator 25 of the calculation device 20 calculates a light intensity Rs obtained by linearly correcting the light intensity As obtained from the detection probe spot SPx on the basis of the following equation (2).

$$Rs = (L2 \times (As/Am1) + L1 \times (As/Am2))/L \ldots (2)$$

In other words, the calculator 25 calculates a ratio between the light intensity As of the detection probe spot SPx and the light intensity Am1 of the marker spot SPa, and a ratio between the light intensity As of the detection probe spot SPx and the light intensity Am2 of the marker spot SPb. Then, the calculator 25 calculates the linearly corrected light intensity Rs by weighting and averaging the calculated light intensity ratio according to the positional relationship between the detection probe spot SPx and the marker spots SPa and SPb.

When the processing described above is completed, in step S16 shown in FIG. 4, the calculator 25 of the calculation device 20 performs processing of measuring the target TG contained in the sample on the basis of the linearly corrected light intensity of the detection probe spot SP1. In this processing, it is determined whether the target TG is contained in the sample added to the nucleic acid sequence measurement device DV using a determination threshold value similar to the first determination threshold value described above.

The calculator 25 may calculate the light intensity by assuming that there is a virtual marker spot at a position of the detection probe spot SPx shown in FIG. 9(a), and may also calculate the light intensity Rs by correcting the light intensity of the detection probe spot SPx using the calculated light intensity of the virtual marker spot. The light intensity Amx of the virtual marker spot at the position of the detection probe spot SPx is calculated using the following equation (3).

$$Amx = (L2 \times Am1 + L1 \times Am2)/L \ldots (3)$$

Furthermore, the light intensity Rs is calculated using the following equation (4).

$$Rs=As/Amx=(As \times L)/(L2 \times Am1+L1 \times Am2)\ldots(4)$$

(b) Spatial correction processing using reference value

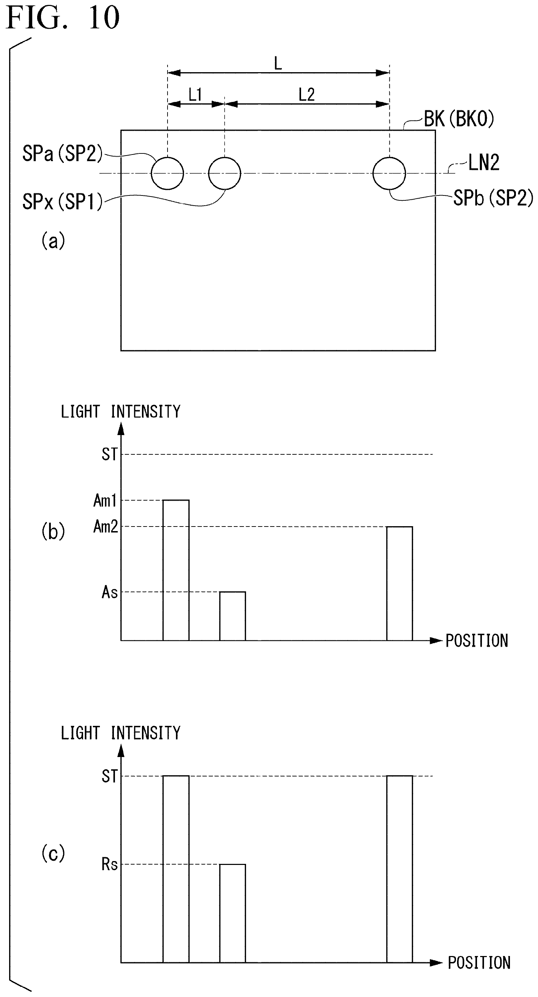

FIG. 10 is a diagram for describing spatial correction processing using a reference value within a block in the embodiment of the present invention. FIG. 10(a) is a diagram which shows a positional relationship of spots in a block BK (the target block BK0), FIG. 10(b) is a diagram which shows light intensities of the spots shown in FIG. 10(a), and FIG. 10(c) is a diagram which shows the light intensities of the spots after correction. Note that FIG. 10(a) is a diagram similar to FIG. 9(a), and FIG. 10(b) is a diagram similar to FIG. 9(b). However, in FIG. 10(b), the reference value is shown as ST.

The ratio between a reference value ST and the light intensity Am1 of the marker spot SPa is k1, and the ratio between the reference value ST and the light intensity Am2 of the marker spot SPb is k2. That is, it is assumed that k1=ST/Am1 and k2=ST/Am2. The calculator 25 of the calculation device 20 calculates the light intensity Rs by linearly correcting the light intensity As obtained from the detection probe spot SPx on the basis of the following equation (5).

$$Rs=As \times (k1+(L1/L) \times (k2-k1)\ldots(5)$$

That is, as shown in FIG. 10(c), the calculator 25 of the calculation device 20 calculates a correction coefficient when correction is performed so that the light intensity Am1 of the marker spot SPa arranged on the straight line LN2 becomes the reference value ST, and the light intensity Am2 of the marker spot SPb becomes the reference value ST. Then, the calculator 25 calculates the light intensity Rs by linearly correcting the light intensity As of the detection probe spot SPx using the correction coefficient according to the positional relationship between the detection probe spot SPx and the marker spots SPa and SPb.

When the processing described above is completed, in step S16 shown in FIG. 4, the calculator 25 of the calculation device 20 performs processing of measuring the target TG contained in the sample on the basis of the linearly corrected light intensity of the detection probe spot SP1. In this processing, it is determined whether the target TG is contained in the sample added to the nucleic acid sequence measurement device DV using a determination threshold value similar to the second determination threshold value described above.

Note that the spatial correction processing within a block BK is not limited to the "spatial correction processing when there is no reference value" and the "spatial correction processing using a reference value." The calculator 25 may calculate a coefficient indicating a change in light intensity of fluorescence along a straight line passing through any two or more marker spots SP2 based on light intensity of fluorescence emitted from any two or more marker spots SP2 among the plurality of marker spots SP2, and correct the spatial distribution of the light intensity of fluorescence emitted from the detection probe spot SP1 positioned on the straight line using the coefficient described above. Alternatively, the calculator 25 may calculate a coefficient indicating a change in light intensity of fluorescence within a block BK based on the light intensity of fluorescence emitted from any three or more marker spots SP2 among the plurality of marker spots SP2, and correct the spatial distribution of the light intensity of fluorescence emitted from any detection probe spot SP1 positioned within a block BK using the coefficient described above.

(c) Spatial correction processing using learning model

The calculator 25 of the calculation device 20 may correct, for example, the light intensity of fluorescence emitted from the detection probe spot SP1 using a learning model generated by machine learning. Here, the learning model described above is a model learned by using information indicating positions of the detection probe spot SP1 and the marker spot SP2 in the image of the target block BK0 and information indicating the light intensity of the marker spot SP2 as explanatory variables, and using a correction coefficient in the detection probe spot SP1 as an objective variable. The calculator 25 of the calculation device 20 corrects the light intensity of fluorescence emitted from the detection probe spot SP1 using a correction coefficient obtained by substituting the explanatory variables described above into the learning model described above.

<<Temporal correction processing>>

(a) Temporal correction processing by normalization

In temporal correction processing using normalization, processing similar to the processing of the flowchart shown in FIG. 6 is performed. For this reason, the following description will be made with reference to FIG. 6. When the processing is started, first, the calculator 25 of the calculation device 20 performs processing of calculating the average value of the light intensities of the marker spot SP2 (step S21). Specifically, processing of calculating the average value of the light intensities of the total of 12 marker spots SP2 in the target block BK0, calculated in step S14 shown in FIG. 4, is performed.

Next, the calculator 25 of the calculation device 20 performs processing of dividing each of the light intensities of the detection probe spots SP1 by the average value of the light intensities of the marker spots SP2 (step S22). Specifically, processing of dividing each of the light intensities of the total of 30 detection probe spots SP1 in the target block BK0, calculated in step S13 shown in FIG. 4, by the average value of the light intensities of the marker spots SP2 is performed. Through this processing, the light intensity of the detection probe spot SP1 that has changed over time is normalized by the average value of the light intensities of the marker spot SP2 that has changed over time, so that the light intensity of detection probe spot SP1 that has changed over time is corrected in the end.

When the processing described above is completed, in step S16 shown in FIG. 4, the calculator 25 of the calculation device 20 performs processing of measuring the target TG contained in the sample on the basis of the normalized light intensity of the detection probe spot SP1. In this processing, it is determined whether the target TG is contained in the sample added to the nucleic acid sequence measurement device DV using a determination threshold value similar to the first determination threshold value described above.

(b) Temporal correction processing using regression formula

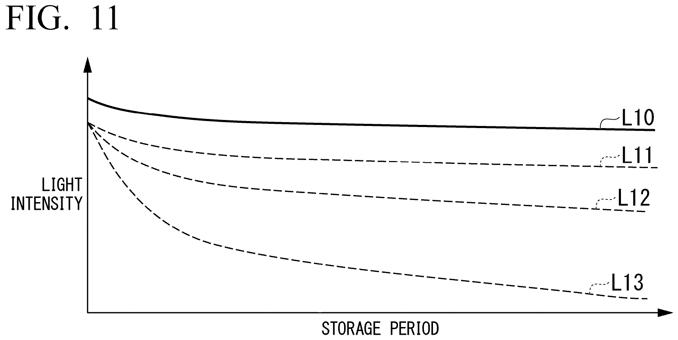

FIG. 11 is a diagram which shows an example of a change over time in spot light intensity. In a graph shown in FIG. 11, the horizontal axis represents a storage period (time) of the nucleic acid sequence measurement device DV, and the vertical axis represents the spot light intensity. In FIG. 11, a curve L10 is a curve showing a change over time in average value of the light intensities of the marker spot SP2 in the target block BK0, and curves L11 to L13 are curves showing changes over time in light intensities of the detection probe spot SP1 in the target block BK0. As shown in FIG. 11, the light intensity of the detection probe spot SP1 changes over time depending on a type of the detection probe spot SP1.

For this reason, data indicating changes over time in light intensity of the detection probe spot SP1 and light intensity of the marker spot SP2 is obtained in advance for each type of the detection probe spot SP1. Then, a regression coefficient is calculated as a deterioration coefficient A by a simple regression analysis using a least square method between the light intensity of the detection probe spot SP1 and the light intensity of the marker spot SP2. This deterioration coefficient A is, for example, stored in advance in the storage 24 of the calculation device 20 in the nuclear acid sequence measurement device 1 shown in FIG. 3. Note that a method of calculating the deterioration coefficient A is not limited to the simple regression analysis. For example, it may be calculated according to inverse proportion, exponential approximation, or logarithmic approximation.

The same processing as processing of the flowchart shown in FIG. 6 is also performed in the temporal correction processing using the regression formula. For this reason, the following description will be made with reference to FIG. 6. When the processing is started, first, the calculator 25 of the calculation device 20 performs processing of calculating the average value of the light intensities of the marker spot SP2 (step S21). Specifically, processing of calculating the average value of the light intensities of the total of 12 marker spots SP2 in the target block BK0, calculated in step S14 shown in FIG. 4 is performed.

Next, the calculator 25 of the calculation device 20 performs processing of dividing each of the light intensities of the detection probe spot SP1 by the average value of the light intensities of the marker spot SP2 (step S22). Specifically, processing of dividing each of the light intensities of the total of 30 detection probe spots SP1 in the target block BK0, calculated in step S13 shown in FIG. 4, by the average value of the light intensities of the marker spots SP2 is performed. Then, the light intensity of the detection probe spot SP1 that has changed over time is corrected by multiplying a value obtained by division by the deterioration coefficient A.

When the processing described above is completed, in step S16 shown in FIG. 4, the calculator 25 of the calculation device 20 performs processing of measuring the target TG contained in the sample on the basis of the normalized light intensity of the detection probe spot SP1. In this processing, it is determined whether the target TG is contained in the sample added to the nucleic acid sequence measurement device DV using a determination threshold value similar to the first determination threshold value described above.

As described above, in the present embodiment, fluorescence emitted from a nucleic acid sequence measurement device DV that is provided with a detection probe spot SP1 that emits fluorescence due to a reaction with the target TG with a specific nucleic acid sequence contained in a sample, and a marker spot SP2 that emits fluorescence regardless of the reaction with the target TG is detected. Then, among the detected fluorescence, the target TG is measured on the basis of a light intensity obtained by correcting or normalizing the light intensity of the fluorescence emitted from the detection probe spot SP1 using the light intensity of the fluorescence emitted from the marker spot SP2. For this reason, even if there is a variation or a change over time in the spot light intensity in the detection probe spot SP1, the target TG can be measured with higher reliability than before.

Although the nucleic acid sequence measurement apparatus, the nucleic acid sequence measurement method, the nucleic acid sequence measurement program, and the nucleic acid sequence measurement device according to the embodiment of the present invention have been described above, the present invention is not limited to the embodiment described above, and can be freely modified within the scope of the present invention. For example, the image acquirer 12a described in the embodiment described above may acquire a monochrome two-dimensional image or a color two-dimensional image.

Further, the image acquirer 12a may include a highly sensitive electron multiplying CCD (MCCD) or a digital CMOS in addition to a CCD and a CMOS. Moreover, the detector 12 may include a photodiode disposed one-to-one with a spot SP instead of the image acquirer 12a (or together with the image acquirer 12a).

In addition, the threshold value used in step S13 shown in FIG. 4 may be changed, or abnormality diagnosis of the nucleic acid sequence measurement device DV may be performed. Hereinafter, details of these will be described in order.

<Change of threshold value used in step S13>

As described using FIG. 5, a spot area is extracted using a threshold value TH0 (a light intensity threshold value) set to a light intensity greater than the background light intensity BG. When a change in spot light intensity occurs over time, it is conceivable that the spot light intensity becomes equal to or less than the threshold value TH0 and an extraction error of a spot area occurs.

Here, the light intensity of the marker spot SP2 is larger than the light intensity of the detection probe spot SP1. For this reason, by correcting the threshold value TH0 on the basis of the change over time in light intensity of the marker spot SP2, the extraction error of a spot area is prevented from occurring. Specifically, first, a regression formula is calculated that indicates the change over time in light intensity of the marker spot SP2 and light intensity of the detection probe spot SP1. Then, according to the regression formula, the light intensity of the detection probe spot SP1 is predicted based on the light intensity of the marker spot SP2 obtained in step S14, and the threshold value TH0 is corrected.

<Abnormality diagnosis of nucleic acid sequence measurement device>

On the basis of the light intensity of the marker spot SP2 acquired in advance, threshold values are set that define upper and lower limits of the light intensity of the marker spot SP2, and the calculator 25 may diagnose an abnormality and notify it when the light intensity of the marker spot S has exceeded the threshold value. The upper limit threshold value can be set, for example, based on errors such as noise or sample-derived abnormal light emission by the image acquirer 12a, and an empirical value of the light intensity of the marker spot SP2.

The lower threshold value is determined by checking a detection limit in advance using the number of target molecules in the sample as a parameter in response to a decrease in light intensity due to deterioration of the detection probe spot SP1 over time, and sets a range of a light intensity that will not be affected by the detection limit in the detection probe spot SP1 with a reduced light intensity. Then, the lower limit threshold can be calculated by calculating a marker spot light intensity in the range that will not be affected by the detection limit of the detection probe.

As a possible range of light intensity correction by the marker spot SP2, it is possible to calculate in advance a range of a marker spot light intensity that deviates from a linearity or nonlinear model of a correlation between the light intensity of the marker spot SP2 and the deterioration of the detection probe spot SP1 over time. The threshold value may be set in a plurality of stages, such as two stages, instead of one stage. When the threshold value is set in two stages, for example, it is possible to set a threshold value that defines that there is a problem with a measured value and a threshold value that defines that the measured value is clearly abnormal.

In addition, the nucleic acid sequence measurement apparatus, the nucleic acid sequence measurement method, the nucleic acid sequence measurement program, and the nucleic acid sequence measurement device according to the embodiment of the present invention can be used for dry image measurement in fluorescent molecules light intensity measurement, in-liquid observation of a fluorescent molecule light intensity of a biochip, and real-time observation in continuous reactions. Specifically, it can be used, for example, to identify bacterial species by a gene and polymer analysis, to identify cancer genes, to identify animals and plants, to examine intestinal bacteria, and the like.

Furthermore, the nucleic acid sequence measurement apparatus and the nucleic acid sequence measurement method according to the embodiment of the present invention are also applied to the following solid phase methods such as a labeled antibody method used in a clinical test and the like. For example, a fluorescence in situ hybridization (FISH) method, which measures fluorescence using a fluorescence substance in an expression of a specific chromosome or gene in a tissue or cell, is cited as an example, In addition, examples of applications include the following various methods. In other words, a fluorescence immunoassay (FIA) method that measures antigen-antibody reactions using a fluorescent substance such as europium as a label, and an indirect fluorescence antibody (IFA) method that measures serum (antibody) reactions obtained by labeling a pathogen and the like, which serve as antigens, with the fluorescent substance.

As used herein, the following directional terms "front, back, above, downward, right, left, vertical, horizontal, below, transverse, row and column" as well as any other similar directional terms refer to those instructions of a device equipped with the present invention. Accordingly, these terms, as utilized to describe the present invention should be interpreted relative to a device equipped with the present invention.

The term "configured" is used to describe a component, unit or part of a device includes hardware and/or software that is constructed and/or programmed to carry out the desired function.

Moreover, terms that are expressed as "means-plus function" in the claims should include any structure that can be utilized to carry out the function of that part of the present invention.

The term "unit" is used to describe a component, unit or part of a hardware and/or software that is constructed and/or programmed to carry out the desired function. Typical examples of the hardware may include, but are not limited to, a device and a circuit.

While preferred embodiments of the present invention have been described and illustrated above, it should be understood that these are examples of the present invention and are not to be considered as limiting. Additions, omissions, substitutions, and other modifications can be made without departing from the scope of the present invention. Accordingly, the present invention is not to be considered as being limited by the foregoing description, and is only limited by the scope of the claims.

**Claims**

1. A nucleic acid sequence measurement apparatus (1) that measures a target (TG) with a specific nucleic acid sequence contained in a sample, the nucleic acid sequence measurement apparatus (1) comprising:

   a detector (12) configured to detect fluorescence emitted from a nucleic acid sequence measurement device (DV), which is provided with a measurement area (SP1) that emits first fluorescence due to a reaction with the target (TG), and a reference area (SP2) that emits second fluorescence regardless of the reaction with the target (TG); and
   a calculator (25) realized by a processor executing a program and configured to measure the target (TG) on the basis of a light intensity obtained by correcting or normalizing a light intensity of the first fluorescence emitted from the measurement area (SP1) using a light intensity of the second fluorescence emitted from the reference area (SP2) in the fluorescence detected by the detector (12),
   **characterized in that**
   the calculator (25) is configured to use information indicating positions of the plurality of reference areas (SP2), information indicating the light intensity of the second fluorescence emitted from the plurality of reference areas (SP2), and information indicating a position of the measurement area (SP1) as explanatory variables, and correct the light intensity of the first fluorescence emitted from the measurement area (SP1) using a correction coefficient obtained by substituting the explanatory variables into a learning model learned using the correction coefficient in the measurement area (SP1) as an objective variable.

2. The nucleic acid sequence measurement apparatus (1) according to claim 1,

   wherein the nucleic acid sequence measurement device (DV) is provided with a plurality of measurement areas (SP1) and the plurality of reference areas (SP2), and
   wherein the calculator (25) is configured to correct or normalize a spatial distribution of a light intensity of the first fluorescence emitted from the plurality of measurement areas (SP1) using a light intensity of the second fluorescence emitted from the plurality of reference areas (SP2).

3. The nucleic acid sequence measurement apparatus (1) according to claim 2,
   wherein the calculator (25) is configured to calculate a coefficient indicating a change in light intensity of the second fluorescence along a straight line (LN2) passing through any two or more reference areas (SP2) based on the light intensity of the second fluorescence emitted from the any two or more of the plurality of reference areas (SP2), and correct the spatial distribution of the light intensity of the first fluorescence emitted from the measurement area (SP1) positioned on the straight line (LN2) using the coefficient.

4. The nucleic acid sequence measurement apparatus (1) according to claim 3,
   wherein the coefficient is a coefficient indicating a rate of change in light intensity of the second fluorescence along the straight line (LN2), or a coefficient based on a multiple regression analysis or a multivariate analysis of the light intensity of the second fluorescence along the straight line (LN2).

5. The nucleic acid sequence measurement apparatus (1) according to claim 1,
   wherein the calculator (25) is configured to correct the light intensity of the first fluorescence emitted from the measurement area (SP1) based on the light intensity of the second fluorescence emitted from the reference area (SP2) using a relational expression showing a relationship between a change over time in light intensity of the first fluorescence emitted from the measurement area (SP1) and a change over time in light intensity of the second fluorescence emitted from the reference area (SP2).

6. The nucleic acid sequence measurement apparatus (1) according to claim 1,
   wherein the calculator (25) is configured to extract the measurement area (SP1) using a predefined light intensity threshold value (THO), and correct the light intensity threshold value (TH0) according to the light intensity of the second fluorescence emitted from the reference area (SP2).

7. The nucleic acid sequence measurement apparatus (1) according to claim 1,

wherein the calculator (25) is configured to notify that there is an abnormality when the light intensity of the second fluorescence emitted from the reference area (SP2) exceeds a preset upper threshold value or does not exceed a preset lower threshold value.

8. The nucleic acid sequence measurement apparatus (1) according to claim 1,

wherein the measurement area (SP1) and the reference area (SP2) in the nucleic acid sequence measurement device (DV) are divided into partition areas (BK) in units of a predetermined number, and

wherein the detector (12) comprises an image acquirer (12a) configured to acquire an image of the partition area.

9. A nucleic acid sequence measurement method that is implemented by a computer and measures a target (TG) with a specific nucleic acid sequence contained in a sample, the nucleic acid sequence measurement method comprising:

a calculation step (S15, S16) of measuring, among fluorescence emitted from a nucleic acid sequence measurement device (DV), which is provided with a measurement area (SP1) that emits first fluorescence due to a reaction with the target (TG), and a reference area (SP2) that emits second fluorescence regardless of the reaction with the target (TG), the target (TG) on the basis of light intensity obtained by correcting or normalizing a light intensity of the first fluorescence emitted from the measurement area (SP1) using light intensity of the second fluorescence emitted from the reference area (SP2),

characterized in that

the calculation step (S15, S16) further comprises a step of using information indicating positions of the plurality of reference areas (SP2), information indicating the light intensity of the second fluorescence emitted from the plurality of reference areas (SP2), and information indicating a position of the measurement area (SP1) as explanatory variables, and correcting the light intensity of the first fluorescence emitted from the measurement area (SP1) using a correction coefficient obtained by substituting the explanatory variables into a learning model learned using the correction coefficient in the measurement area (SP1) as an objective variable.

10. A nucleic acid sequence measurement program that causes a computer to execute:

a calculation step (S15, S16) of measuring, among fluorescence emitted from a nucleic acid sequence measurement device (DV), which is provided with a measurement area (SP1) that emits first fluorescence due to a reaction with the target (TG), and a reference area (SP2) that emits second fluorescence regardless of the reaction with the target (TG), the target (TG) on the basis of light intensity obtained by correcting or normalizing a light intensity of the first fluorescence emitted from the measurement area (SP1) using a light intensity of the second fluorescence emitted from the reference area (SP2),

characterized in that

the calculation step (S15, S16) further comprises a step of using information indicating positions of the plurality of reference areas (SP2), information indicating the light intensity of the second fluorescence emitted from the plurality of reference areas (SP2), and information indicating a position of the measurement area (SP1) as explanatory variables, and correcting the light intensity of the first fluorescence emitted from the measurement area (SP1) using a correction coefficient obtained by substituting the explanatory variables into a learning model learned using the correction coefficient in the measurement area (SP1) as an objective variable.

**Patentansprüche**

1. Vorrichtung (1) zur Messung von Nukleinsäuresequenzen, die ein Zielobjekt (TG) mit einer spezifischen Nukleinsäuresequenz misst, die in einer Probe enthalten ist, wobei die Vorrichtung (1) zur Messung von Nukleinsäuresequenzen umfasst:

einen Detektor (12), der so konfiguriert ist, dass er Fluoreszenz erfasst, die von einem Nukleinsäuresequenz-Messgerät (DV) emittiert wird, das mit einem Messbereich (SP1) versehen ist, der aufgrund einer Reaktion mit dem Zielobjekt (TG) eine erste Fluoreszenz emittiert, und einem Referenzbereich (SP2), der unabhängig von der Reaktion mit dem Zielobjekt (TG) eine zweite Fluoreszenz emittiert; und

einen Rechner (25), der durch einen Prozessor realisiert ist, der ein Programm ausführt, und der so konfiguriert ist, dass er das Zielobjekt (TG) auf der Grundlage einer Lichtintensität misst, die durch Korrigieren oder Normalisieren einer Lichtintensität der ersten Fluoreszenz, die von dem Messbereich (SP1) emittiert wird, unter Verwendung einer Lichtintensität der zweiten Fluoreszenz, die von dem Referenzbereich (SP2) in der von dem Detektor (12) erfassten Fluoreszenz emittiert wird, erhalten wird, **dadurch gekennzeichnet, dass** der Rechner (25) so konfiguriert ist, dass er Informationen, die Positionen der mehreren Referenzbereiche (SP2) angeben, Informationen, die die Lichtintensität der zweiten Fluoreszenz angeben, die von den mehreren Referenzbereichen (SP2) emittiert wird, und Informationen, die eine Position des Messbereichs (SP1) angeben, als erklärende Variablen verwendet und die Lichtintensität der ersten Fluoreszenz, die aus dem Messbereich (SP1) emittiert wird, unter Verwendung eines Korrekturkoeffizienten korrigiert, der durch Einsetzen der erklärenden Variablen in ein Lernmodell erhalten wird, das unter Verwendung des Korrekturkoeffizienten im Messbereich (SP1) als Zielvariable gelernt wurde.

2. Die Vorrichtung (1) zur Messung von Nukleinsäuresequenzen gemäß Anspruch 1,

   wobei das Nukleinsäuresequenz-Messgerät (DV) mit mehreren Messbereichen (SP1) und mehreren Referenzbereichen (SP2) versehen ist, und
   wobei der Rechner (25) so konfiguriert ist, dass er eine räumliche Verteilung einer Lichtintensität der ersten Fluoreszenz, die von den mehreren Messbereichen (SP1) emittiert wird, unter Verwendung einer Lichtintensität der zweiten Fluoreszenz, die von den mehreren Referenzbereichen (SP2) emittiert wird, korrigiert oder normalisiert.

3. Die Vorrichtung (1) zur Messung von Nukleinsäuresequenzen gemäß Anspruch 2, wobei der Rechner (25) so konfiguriert ist, dass er einen Koeffizienten berechnet, der eine Änderung der Lichtintensität der zweiten Fluoreszenz entlang einer geraden Linie (LN2) angibt, die durch zwei oder mehr beliebige Referenzbereiche (SP2) verläuft, basierend auf der Lichtintensität der zweiten Fluoreszenz, die von zwei oder mehr beliebige der mehreren Referenzbereiche (SP2) emittiert wird, und die räumliche Verteilung der Lichtintensität der ersten Fluoreszenz, die von dem auf der geraden Linie (LN2) positionierten Messbereich (SP1) emittiert wird, unter Verwendung des Koeffizienten korrigiert.

4. Die Vorrichtung (1) zur Messung von Nukleinsäuresequenzen gemäß Anspruch 3, wobei der Koeffizient ein Koeffizient ist, der eine Änderungsrate der Lichtintensität der zweiten Fluoreszenz entlang der geraden Linie (LN2) angibt, oder ein Koeffizient ist, der auf einer multiplen Regressionsanalyse oder einer multivariaten Analyse der Lichtintensität der zweiten Fluoreszenz entlang der geraden Linie (LN2) basiert.

5. Die Vorrichtung (1) zur Messung von Nukleinsäuresequenzen gemäß Anspruch 1, wobei der Rechner (25) so konfiguriert ist, dass er die Lichtintensität der ersten Fluoreszenz, die aus dem Messbereich (SP1) auf der Grundlage der Lichtintensität der zweiten Fluoreszenz, die aus dem Referenzbereich (SP2) emittiert wird, unter Verwendung eines relationalen Ausdrucks korrigiert, der eine Beziehung zwischen einer zeitlichen Änderung der Lichtintensität der ersten Fluoreszenz, die aus dem Messbereich (SP1) emittiert wird, und einer zeitlichen Änderung der Lichtintensität der zweiten Fluoreszenz, die aus dem Referenzbereich (SP2) emittiert wird, zeigt.

6. Die Vorrichtung (1) zur Messung von Nukleinsäuresequenzen gemäß Anspruch 1, wobei der Rechner (25) so konfiguriert ist, dass er den Messbereich (SP1) unter Verwendung eines vordefinierten Lichtintensitätsschwellenwerts (TH0) extrahiert und den Lichtintensitätsschwellenwert (TH0) entsprechend der Lichtintensität der zweiten Fluoreszenz, die aus dem Referenzbereich (SP2) emittiert wird, korrigiert.

7. Die Vorrichtung (1) zur Messung von Nukleinsäuresequenzen gemäß Anspruch 1, wobei der Rechner (25) so konfiguriert ist, dass er eine Anomalie meldet, wenn die Lichtintensität der zweiten Fluoreszenz, die aus dem Referenzbereich (SP2) emittiert wird, einen voreingestellten oberen Schwellenwert überschreitet oder einen voreingestellten unteren Schwellenwert nicht überschreitet.

8. Die Vorrichtung (1) zur Messung von Nukleinsäuresequenzen gemäß Anspruch 1,

   wobei der Messbereich (SP1) und der Referenzbereich (SP2) in dem Nukleinsäuresequenz-Messgerät (DV) in Partitionsbereiche (BK) in Einheiten einer vorbestimmten Anzahl unterteilt sind, und
   wobei der Detektor (12) einen Bildaufnehmer (12a) umfasst, der so konfiguriert ist, dass er ein Bild des Teilungsbereichs aufnimmt.

9. Verfahren zur Messung von Nukleinsäuresequenzen, das von einem Computer ausgeführt wird und ein Zielobjekt (TG) mit einer bestimmten Nukleinsäuresequenz, das in einer Probe enthalten ist, misst, wobei das Verfahren zur Messung von Nukleinsäuresequenzen umfasst:

   einen Berechnungsschritt (S15, S16) zum Messen, unter der von einem Nukleinsäuresequenz-Messgerät (DV) emittierten Fluoreszenz, die mit einem Messbereich (SP1) versehen ist, der aufgrund einer Reaktion mit dem Zielobjekt (TG) eine erste Fluoreszenz emittiert, und einem Referenzbereich (SP2), der unabhängig von der Reaktion mit dem Zielobjekt (TG) eine zweite Fluoreszenz emittiert, das Zielobjekt (TG) auf der Grundlage der Lichtintensität zu messen, die durch Korrigieren oder Normalisieren einer Lichtintensität der ersten Fluoreszenz, die von dem Messbereich (SP1) emittiert wird, unter Verwendung der Lichtintensität der zweiten Fluoreszenz, die von dem Referenzbereich (SP2) emittiert wird, erhalten wird,

**dadurch gekennzeichnet, dass**

der Berechnungsschritt (S15, S16) ferner einen Schritt umfasst, bei dem Informationen, die Positionen der mehreren Referenzbereiche (SP2) angeben, Informationen, die die Lichtintensität der von den mehreren Referenzbereichen (SP2) emittierten zweiten Fluoreszenz angeben, und Informationen, die eine Position des Messbereichs (SP1) angeben, als erklärende Variablen verwendet werden und die Lichtintensität der ersten Fluoreszenz, die aus dem Messbereich (SP1) emittiert wird, unter Verwendung eines Korrekturkoeffizienten korrigiert wird, der durch Einsetzen der erklärenden Variablen in ein Lernmodell erhalten wird, das unter Verwendung des Korrekturkoeffizienten im Messbereich (SP1) als Zielvariable gelernt wurde.

**10.** Nukleinsäuresequenz-Messprogramm, das einen Computer dazu veranlasst, Folgendes auszuführen:

einen Berechnungsschritt (S15, S16) zum Messen, unter der Fluoreszenz, die von einem Nukleinsäuresequenz-Messgerät (DV) emittiert wird, das mit einem Messbereich (SP1) versehen ist, der eine erste Fluoreszenz aufgrund einer Reaktion mit dem Zielobjekt (TG) emittiert, und einem Referenzbereich (SP2), der eine zweite Fluoreszenz unabhängig von der Reaktion mit dem Zielobjekt (TG) emittiert, das Zielobjekt (TG) auf der Grundlage der Lichtintensität, die durch Korrigieren oder Normalisieren einer Lichtintensität der ersten Fluoreszenz, die von dem Messbereich (SP1) emittiert wird, unter Verwendung einer Lichtintensität der zweiten Fluoreszenz, die von dem Referenzbereich (SP2) emittiert wird, erhalten wird,

**dadurch gekennzeichnet, dass**

der Berechnungsschritt (S15, S16) ferner einen Schritt umfasst, bei dem Informationen, die Positionen der mehreren Referenzbereiche (SP2) angeben, Informationen, die die Lichtintensität der von den mehreren Referenzbereichen (SP2) emittierten zweiten Fluoreszenz angeben, und Informationen, die eine Position des Messbereichs (SP1) angeben, als erklärende Variablen verwendet und die Lichtintensität der ersten Fluoreszenz, die aus dem Messbereich (SP1) emittiert wird, unter Verwendung eines Korrekturkoeffizienten korrigiert, der durch Einsetzen der erklärenden Variablen in ein Lernmodell erhalten wird, das unter Verwendung des Korrekturkoeffizienten im Messbereich (SP1) als Zielvariable gelernt wurde.

**Revendications**

**1.** Appareil de mesure de séquence d'acide nucléique (1) qui mesure une cible (TG) avec une séquence d'acide nucléique spécifique contenue dans un échantillon, l'appareil de mesure de séquence d'acide nucléique (1) comprenant:

un détecteur (12) configuré pour détecter la fluorescence émise par un dispositif de mesure de séquence d'acide nucléique (DV), qui est muni d'une zone de mesure (SP1) qui émet une première fluorescence due à une réaction avec la cible (TG), et d'une zone de référence (SP2) qui émet une seconde fluorescence indépendamment de la réaction avec la cible (TG); et un calculateur (25) réalisé par un processeur exécutant un programme et configuré pour mesurer la cible (TG) sur la base d'une intensité lumineuse obtenue en corrigeant ou en normalisant une intensité lumineuse de la première fluorescence émise depuis la zone de mesure (SP1) à l'aide d'une intensité lumineuse de la seconde fluorescence émise depuis la zone de référence (SP2) dans la fluorescence détectée par le détecteur (12),

**caractérisé en ce que**

le calculateur (25) est configuré pour utiliser des informations indiquant les positions de la pluralité de zones de référence (SP2), des informations indiquant l'intensité lumineuse de la seconde fluorescence émise à partir de la pluralité de zones de référence (SP2), et des informations indiquant une position de la zone de mesure (SP1) comme variables explicatives, et corriger l'intensité lumineuse de la première fluorescence émise depuis la zone de mesure (SP1) en utilisant un coefficient de correction obtenu en substituant les variables explicatives dans un modèle d'apprentissage appris en utilisant le coefficient de correction dans la zone de mesure (SP1) comme variable objective.

**2.** Appareil de mesure de séquence d'acide nucléique (1) selon la revendication 1,

dans lequel le dispositif de mesure de séquence d'acide nucléique (DV) est muni d'une pluralité de zones de mesure (SP1) et de la pluralité de zones de référence (SP2), et

dans lequel le calculateur (25) est configuré pour corriger ou normaliser une distribution spatiale d'une intensité lumineuse de la première fluorescence émise à partir de la pluralité de zones de mesure (SP1) en utilisant une intensité lumineuse de la seconde fluorescence émise à partir de la pluralité de zones de référence (SP2).

3. Appareil de mesure de séquence d'acide nucléique (1) selon la revendication 2, dans lequel le calculateur (25) est configuré pour calculer un coefficient indiquant un changement d'intensité lumineuse de la seconde fluorescence le long d'une ligne droite (LN2) passant par deux ou plusieurs zones de référence (SP2) quelconques sur la base de l'intensité lumineuse de la seconde fluorescence émise à partir de deux ou plusieurs zones quelconques parmi la pluralité de zones de référence (SP2), et corriger la distribution spatiale de l'intensité lumineuse de la première fluorescence émise par la zone de mesure (SP1) positionnée sur la ligne droite (LN2) à l'aide du coefficient.

4. Appareil de mesure de séquence d'acide nucléique (1) selon la revendication 3, dans lequel le coefficient est un coefficient indiquant un taux de variation de l'intensité lumineuse de la seconde fluorescence le long de la ligne droite (LN2), ou un coefficient reposant sur une analyse de régression multiple ou une analyse multivariée de l'intensité lumineuse de la seconde fluorescence le long de la ligne droite (LN2).

5. Appareil de mesure de séquence d'acide nucléique (1) selon la revendication 1, dans lequel le calculateur (25) est configuré pour corriger l'intensité lumineuse de la première fluorescence émise depuis la zone de mesure (SP1) sur la base de l'intensité lumineuse de la seconde fluorescence émise depuis la zone de référence (SP2) à l'aide d'une expression relationnelle montrant une relation entre une variation dans le temps de l'intensité lumineuse de la première fluorescence émise depuis la zone de mesure (SP1) et une variation dans le temps de l'intensité lumineuse de la seconde fluorescence émise depuis la zone de référence (SP2).

6. Appareil de mesure de séquence d'acide nucléique (1) selon la revendication 1,
dans lequel le calculateur (25) est configuré pour extraire la zone de mesure (SP1) à l'aide d'une valeur seuil d'intensité lumineuse prédéfinie (THO), et corriger la valeur seuil d'intensité lumineuse (THO) en fonction de l'intensité lumineuse de la seconde fluorescence émise depuis la zone de référence (SP2).

7. Appareil de mesure de séquence d'acide nucléique (1) selon la revendication 1, dans lequel le calculateur (25) est configuré pour signaler qu'il y a une anomalie lorsque l'intensité lumineuse de la seconde fluorescence émise par la zone de référence (SP2) dépasse une valeur seuil supérieure prédéfinie ou ne dépasse pas une valeur seuil inférieure prédéfinie.

8. Appareil de mesure de séquence d'acide nucléique (1) selon la revendication 1, dans lequel la zone de mesure (SP1) et la zone de référence (SP2) dans le dispositif de mesure de séquence d'acide nucléique (DV) sont divisées en zones de partition (BK) par unités d'un nombre prédéterminé, et
dans lequel le détecteur (12) comprend un dispositif d'acquisition d'images (12a) configuré pour acquérir une image de la zone de partition.

9. Procédé de mesure de séquence d'acide nucléique qui est mis en œuvre par un ordinateur et mesure une cible (TG) avec une séquence d'acide nucléique spécifique contenue dans un échantillon, le procédé de mesure de séquence d'acide nucléique comprenant:

une étape de calcul (S15, S16) consistant à mesurer, parmi la fluorescence émise par un dispositif de mesure de séquence d'acide nucléique (DV), qui est muni d'une zone de mesure (SP1) qui émet une première fluorescence due à une réaction avec la cible (TG), et d'une zone de référence (SP2) qui émet une seconde fluorescence indépendamment de la réaction avec la cible (TG), la cible (TG) sur la base de l'intensité lumineuse obtenue en corrigeant ou en normalisant l'intensité lumineuse de la première fluorescence émise par la zone de mesure (SP1) à l'aide de l'intensité lumineuse de la seconde fluorescence émise par la zone de référence (SP2),
**caractérisé en ce que**
l'étape de calcul (S15, S16) comprend en outre une étape consistant à utiliser des informations indiquant les positions de la pluralité de zones de référence (SP2), des informations indiquant l'intensité lumineuse de la seconde fluorescence émise à partir de la pluralité de zones de référence (SP2), et des informations indiquant une position de la zone de mesure (SP1) comme variables explicatives, et la correction de l'intensité lumineuse de la première fluorescence émise depuis la zone de mesure (SP1) à l'aide d'un coefficient de correction obtenu en substituant les variables explicatives dans un modèle d'apprentissage par l' e appris à l'aide du coefficient de correction dans la zone de mesure (SP1) comme variable objective.

10. Programme de mesure de séquence d'acide nucléique qui conduit un ordinateur à exécuter:

une étape de calcul (S15, S16) consistant à mesurer, parmi la fluorescence émise par un dispositif de mesure de séquence d'acide nucléique (DV) qui est muni d'une zone de mesure (SP1) qui émet une première fluorescence due à une réaction avec la cible (TG), la cible (TG) sur la base de l'intensité lumineuse obtenue en corrigeant ou en normalisant une intensité lumineuse de la première fluorescence émise par la zone de mesure (SP1) et d'une zone de référence (SP2) qui émet une seconde fluorescence indépendamment de la réaction avec la cible (TG), la cible (TG) sur la base de l'intensité lumineuse obtenue en corrigeant ou en normalisant une intensité lumineuse de la seconde fluorescence (TG), et une zone de référence (SP2) qui émet une seconde fluorescence indépendamment de la réaction avec la cible (TG), la cible (TG) sur la base de l'intensité lumineuse obtenue en corrigeant ou en normalisant l'intensité lumineuse de la première fluorescence émise par la zone de mesure (SP1) à l'aide de l'intensité lumineuse de la seconde fluorescence émise par la zone de référence (SP2), **caractérisé en ce que** l'étape de calcul (S15, S16) comprend en outre une étape consistant à utiliser des informations indiquant les positions de la pluralité de zones de référence (SP2), des informations indiquant l'intensité lumineuse de la seconde fluorescence émise à partir de la pluralité de zones de référence (SP2), et des informations indiquant une position de la zone de mesure (SP1) en tant que variables explicatives, et la correction de l'intensité lumineuse de la première fluorescence émise depuis la zone de mesure (SP1) à l'aide d'un coefficient de correction obtenu en substituant les variables explicatives dans un modèle d'apprentissage appris à l'aide du coefficient de correction dans la zone de mesure (SP1) en tant que variable objective.

# FIG. 1

FIG. 2

FIG. 3

# FIG. 4

```
                    ┌─────────┐
                    │  START  │
                    └─────────┘
                         │
                         ▼                        ╭S11
    ┌──────────────────────────────────────────────┐
    │      ACQUIRE  IMAGE  AFTER  SAMPLE  ADDITION   │
    └──────────────────────────────────────────────┘
                         │
                         ▼                        ╭S12
    ┌──────────────────────────────────────────────┐
    │              EXTRACT  SPOT  AREA              │
    └──────────────────────────────────────────────┘
                         │
                         ▼                        ╭S13
    ┌──────────────────────────────────────────────┐
    │  CALCULATE  LIGHT  INTENSITY  OF  DETECTION  PROBE  SPOT │
    └──────────────────────────────────────────────┘
                         │
                         ▼                        ╭S14
    ┌──────────────────────────────────────────────┐
    │   CALCULATE  LIGHT  INTENSITY  OF  MARKER  SPOT │
    └──────────────────────────────────────────────┘
                         │
                         ▼                        ╭S15
    ┌──────────────────────────────────────────────┐
    │ USE LIGHT INTENSITY OF MARKER SPOT TO NORMALIZE OR │
    │ CORRECT LIGHT INTENSITY OF DETECTION PROBE SPOT │
    └──────────────────────────────────────────────┘
                         │
                         ▼                        ╭S16
    ┌──────────────────────────────────────────────┐
    │              MEASURE  TARGET                  │
    └──────────────────────────────────────────────┘
                         │
                         ▼
                    ┌─────────┐
                    │   END   │
                    └─────────┘
```

# FIG. 5

(a)  SP1

LN1

(b)

THO  BG

0

# FIG. 6

```
        START
          │
          ▼                          S21
┌────────────────────────────────────────┐
│ CALCULATE AVERAGE VALUE OF LIGHT         │
│ INTENSITIES OF MARKER SPOT               │
└────────────────────────────────────────┘
          │
          ▼                          S22
┌────────────────────────────────────────┐
│ DIVIDE EACH LIGHT INTENSITY OF DETECTION │
│ PROBE SPOT BY AVERAGE VALUE OF LIGHT     │
│ INTENSITIES OF MARKER SPOT AND           │
│ NORMALIZE THEM                           │
└────────────────────────────────────────┘
          │
          ▼
         END
```

# FIG. 7

```
        START
          │
          ▼                          S31
┌────────────────────────────────────────┐
│ CALCULATE AVERAGE VALUE OF LIGHT         │
│ INTENSITIES OF MARKER SPOT               │
└────────────────────────────────────────┘
          │
          ▼                          S32
┌────────────────────────────────────────┐
│ CALCULATE CORRECTION COEFFICIENT         │
│ INDICATING DEVIATION BETWEEN AVERAGE     │
│ VALUE AND REFERENCE VALUE OF LIGHT       │
│ INTENSITIES OF MARKER SPOT               │
└────────────────────────────────────────┘
          │
          ▼                          S33
┌────────────────────────────────────────┐
│ PERFORM CORRECTION BY MULTIPLYING EACH   │
│ OF LIGHT INTENSITIES OF DETECTION PROBE  │
│ SPOT BY CORRECTION COEFFICIENT           │
└────────────────────────────────────────┘
          │
          ▼
         END
```

# FIG. 8

START

↓

CALCULATE ASSUMED REFERENCE VALUE ⌐S41

↓

CALCULATE AVERAGE VALUE OF LIGHT INTENSITIES OF MARKER SPOT ⌐S42

↓

CALCULATE CORRECTION COEFFICIENT INDICATING DEVIATION BETWEEN AVERAGE VALUE AND ASSUMED REFERENCE VALUE OF LIGHT INTENSITIES OF MARKER SPOT ⌐S43

↓

PERFORM CORRECTION BY MULTIPLYING EACH OF LIGHT INTENSITIES OF DETECTION PROBE SPOT BY CORRECTION COEFFICIENT ⌐S44

↓

END

# FIG. 9

# FIG. 10

(a)

L

L1    L2

BK (BK0)

SPa (SP2)

LN2

SPx (SP1)

SPb (SP2)

(b)

LIGHT INTENSITY

ST

Am1
Am2

As

POSITION

(c)

LIGHT INTENSITY

ST

Rs

POSITION

FIG. 11

LIGHT INTENSITY

STORAGE PERIOD

L10

L11

L12

L13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023083113 A
- JP 5928906 B
- GB 2435473 A